(19) Europäisches Patentamt
European Patent Office
Office européen des brevets

(11) Veröffentlichungsnummer : **0 213 290**
**B1**

(12) # EUROPÄISCHE PATENTSCHRIFT

(45) Veröffentlichungstag der Patentschrift : **07.09.88**

(51) Int. Cl.⁴ : **C 07 C102/00**, C 07 C103/127

(21) Anmeldenummer : **86107780.8**

(22) Anmeldetag : **07.06.86**

(54) **Verfahren zur Herstellung von N- -Alkoxy-ethyl-formaimiden.**

(30) Priorität : **11.06.85 DE 3520829**

(43) Veröffentlichungstag der Anmeldung : **11.03.87 Patentblatt 87/11**

(45) Bekanntmachung des Hinweises auf die Patenterteilung : **07.09.88 Patentblatt 88/36**

(84) Benannte Vertragsstaaten : **DE FR GB IT**

(56) Entgegenhaltungen :
**DE-A- 2 944 456**
**FR-A- 2 558 156**
**US-A- 4 492 619**

(73) Patentinhaber : **BASF Aktiengesellschaft**
**Carl-Bosch-Strasse 38**
**D-6700 Ludwigshafen (DE)**

(72) Erfinder : **Oftring, Alfred, Dr.**
**Berner Weg 26**
**D-6700 Ludwigshafen (DE)**
Erfinder : **Hahn, Erwin, Dr.**
**Am Buechsenackerhang 31**
**D-6900 Heidelberg (DE)**
Erfinder : **Fikentscher, Rolf, Dr.**
**Von-Stephan-Strasse 27**
**D-6700 Ludwigshafen (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 213 290 B1

**Beschreibung**

Aus der EP-PS 19 226 ist ein Verfahren zur Herstellung von N-α-Alkoxy-ethyl-carbonsäureamiden bekannt, bei dem man N-Ethyl-carbonsäureamide mit einem Alkohol in einer Elektrolysezelle in Gegenwart eines Leitsalzes anodisch alkoxyliert. Das elektrochemische Verfahren erscheint sowohl von der Verfahrensweise als auch von den Einsatzstoffkosten her gesehen als recht aufwendig.

Der vorliegenden Erfindung liegt die Aufgabe zugrunde, ein anderes vorteilhafteres Verfahren zur Herstellung von N-α-Alkoxy-ethyl-formamiden zur Verfügung zu stellen.

Die Aufgabe wird erfindungsgemäß dadurch gelöst, daß man einen Vinylether der Formel

$$CH_2{=}CH{-}OR \qquad (II)$$

in der R=$C_1$- bis $C_{18}$-Alkyl bedeutet, in Gegenwart eines sauren oder basischen Katalysators bei Temperaturen von — 10 bis 150 °C mit Formamid in einem molaren Verhältnis von 20 : 1 bis 1 : 2 umsetzt, wobei die Menge des Katalysators 0,1 bis 10 Gew. %, bezogen auf Vinylether und Formamid, beträgt. Vorzugsweise verwendet man solche Verbindungen der Formel II, in der R eine $C_1$- bis $C_4$-Alkylgruppe bedeutet, nämlich, Vinylmethylether, Vinyl-ethylether, Vinyl-n-propylether, Vinyl-isopropylether, Vinyl-n-butyl-ether, Vinyl-isobutylether und Vinyl-tert.-butylether.

Für das erfindungsgemäße Verfahren eignen sich außerdem Vinylalkylether, in denen der Alkylrest bis zu 18 C-Atome enthält, z. B. Vinylhexylether, Vinyl-2-ethylhexylether, Vinylisooctylether, Vinylnonylether, Vinyldodecylether, Vinylpalmitylether und Vinylstearylether.

Die Vinylether der Formel II werden mit Formamid gemäß folgendem Schema

$$\underset{(II)}{CH_2 = CH - OR} + H - CO - NH_2 \longrightarrow CH_3 - \overset{\displaystyle OR}{\overset{|}{CH}} - NH - CHO \qquad (I)$$

in Gegenwart von sauren oder basischen Katalysatoren bei Temperaturen von — 10 bis 150, vorzugsweise 0 bis 70 °C umgesetzt. Der Substituent R in den Formeln I und II steht für eine $C_1$- bis $C_{18}$-Alkylgruppe. Die Verbindungen der Formel II werden mit Formamid in einem molaren Verhältnis von 20 : 1 bis 1 : 2, vorzugsweise 3 : 1 bis 1 : 1 umgesetzt. Ein Überschuß an Vinylalkylether wirkt als Verdünnungsmittel.

Die Umsetzung kann auch gegebenenfalls in einem polaren Lösemittel vorgenommen werden, das unter den Reaktionsbedingungen inert ist. Geeignete Lösemittel sind beispielsweise Diethylether, Tetrahydrofuran, Dioxan, Dimethylformamid, Dimethylglykolether, Diethylglykolether, Methylglykol, Ethylglykol, Polyethylenglykole, die 2 bis 15 Ethylenoxid-Einheiten enthalten, Propylenglykol, bei Raumtemperatur flüssige Polypropylenglykole, die 2 bis 15 Propylenoxid-Einheiten enthalten, bei 20 °C flüssige Blockcopolymerisate aus Ethylenoxid und Propylenoxid, $C_1$- bis $C_4$-Alkohole, wie Methanol, Ethanol, Isopropanol, n-Propanol, n-Butanol, iso-Butanol und tert.-Butanol, sowie Acetonitril. Für den Fall, daß man die Umsetzung in einem inerten Lösemittel vornimmt, verwendet man pro 100 Gewichtsteile der Mischung aus Vinylether und Formamid bis zu 1000, vorzugsweise 10 bis 200 Gewichtsteile eines inerten Lösungsmittels oder einer Mischung mehrerer inerter Lösemittel. Das jeweils in Betracht kommende Lösemittel ist den Katalysatoren anzupassen, d. h. die Lösemittel müssen unter den Reaktionsbedingungen inert sein.

Die Umsetzung der beiden oben beschriebenen Reaktionspartner erfolgt nur in Gegenwart eines sauren oder eines basischen Katalysators. Als saure Katalysatoren eignen sich starke Säuren, deren $pK_a$-Wert höchtens 2 beträgt, z. B. Schwefelsäure, Amidosulfonsäure, aliphatische und aromatische Sulfonsäuren, wie Trifluormethansulfonsäure, Methansulfonsäure, Benzolsulfonsäure, p-Toluolsulfonsäure sowie Lewis-Säuren, z. B. Bortrifluorid-diethyletherat, Zinn-(IV)-chlorid, $AlBr_3$, $FeBr_2$, $FeCl_3$, $ZnCl_2$, $AlCl_3$, $TiCl_4$, und $CuCl_2$. Man kann auch Gemische verschiedener Säuren bzw. Mischungen aus Schwefelsäure und einer Lewis-Säure, z. B. $BF_3$-Etherat, verwenden.

Geeignete basische Katalysatoren sind z. B. tertiäre Amine, wie Triethylamin, Triethanolamin, Dimethylhexylamin, Pyridin, Chinolin, Chinolidin, 1,4-Diazabicyclo-[2,2,2]-octan und Pyrrocolin. Es ist selbstverständlich auch möglich, Mischungen aus verschiedenen basischen Katalysatoren einzusetzen.

Die sauren bzw. basischen Katalysatoren werden, bezogen auf den eingesetzten Vinylether und Formamid, in einer Menge von 0,1 bis 10, vorzugsweise 0,5 bis 5 Gew. % bei der Umsetzung verwendet. Vorzugsweise setzt man saure Katalysatoren ein und führt die Umsetzung in dem Temperaturbereich von 0 bis 70 °C durch. Als Verbindung der Formel II werden vor allem Vinylmethylether und Vinylethylether mit Formamid im Molverhältnis 3 : 1 bis 1 : 1 in Gegenwart von 0,5 bis 5 Gew. % Bortrifluoriddiethyletherat als sauren Katalysator eingesetzt. Die Reaktionen werden vorzugsweise unter Ausschluß von Sauerstoff in einer Inertgasatmosphäre durchgeführt. Als Inertgas kommt hauptsächlich Stickstoff in Betracht. Die Umsetzung erfolgt vorzugsweise in wasserfreiem Medium bzw. unter weitgehendem Auschluß von

Wasser, jedoch kann bis zu etwa 1 Gew.% Wasser, bezogen auf Vinylether und Formamid, im Reaktionsgemisch vorhanden sein. Man kann die Umsetzung bei Normaldruck, unter erhöhtem Druck, z. B. bis zu 100 bar, oder unter vermindertem Druck durchführen.

Nach Beendigung der Umsetzung wird das Reaktionsgemisch gereinigt. Um den eingesetzten Katalysator zu entfernen, wird eine Neutralisation durchgeführt. Sofern man einen sauren Katalysator verwendet hat, gibt man der Einfachheit halber Soda zum Reaktionsgemisch, trennt überschüssige Soda und das bei der Neutralisation jeweils entstehende Salz ab, z. B. durch Filtrieren, und destilliert das Filtrat.

Aus den so hergestellten N-α-Alkoxy-ethyl-formamiden der Formel I wird durch Pyrolyse unter Abspaltung von R-OH, wobei R die in Formel I angegebene Bedeutung hat, N-Vinylformamid hergestellt. N-Vinylformamid ist ein wertvolles Monomer, das z. B. durch Homopolymerisation und anschließende partielle Verseifung zu hochwirksamen kationischen Flockungsmitteln sowie Retentionsmitteln und Entwässerungshilfsmitteln bei der Papierherstellung verarbeitet werden kann. Die Pyrolyse der Verbindungen der Formel II erfolgt bei Normaldruck oder vorzugsweise unter vermindertem Druck, z. B. in dem Bereich von 5 bis 500, insbesondere 10 bis 200 mbar, bei Temperaturen von 200 bis 650, vorzugsweise 300 bis 550 °C. Die Pyrolyse kann an Feststoffen als Katalysator vorgenommen werden, z. B. $SiO_2$, Tonerde, $Al_2O_3$, Marmor, Eisen, Kupfer, MgO und ZnO sowie Mischungen von geeigneten Feststoffen. Die Katalysatoren können in Form von Ringen, Kugeln, Strängen oder Hohlkörpern vorliegen.

Die in den Beispielen angegebenen Teile sind Gewichtsteile.

## Beispiel 1

In einem Kolben wird eine Lösung von 58 g (1 Mol) Vinylmethylether in 40 g Tetrahydrofuran vorgelegt und auf eine Temperatur von 5 °C abgekühlt. Anschließend gibt man innerhalb von 6 Stunden eine Lösung aus 45 g (1 Mol) Formamid, 1,5 g (0,01 Mol) Bortrifluoriddiethyletherat und 30 g Tetrahydrofuran bei einer Reaktionstemperatur im Bereich von 0 bis 5 °C unter kräftigem Rühren zu. Die Reaktion wird unter Ausschluß von Sauerstoff in einer Stickstoffatmosphäre durchgeführt. Danach rührt man das Reaktionsgemisch 12 Stunden bei Raumteperatur, setzt 5 g feste Soda zu, filtriert und destilliert das als Lösemittel verwendete Tetrahydrofuran und andere flüchtige Anteile im Wasserstrahlvakuum bei einer Temperatur von 25 °C ab. Es verbleibt ein öliger Rückstand, der dann über eine 30 cm lange Kolonne fraktioniert destilliert wird. Man erhält 65 g (63 % der Theorie) N-α-Methoxy-ethyl-formamid in Form einer farblosen Flüssigkeit vom Siedepunkt 47 bis 49 °C bei einem Druck von 0,2 mbar.

## Beispiel 2

Zu einer Lösung von 72 g (1,2 Mol) Vinylmethylether in 70 g Acetonitril, die eine Temperatur von 5 °C aufweist, wird unter kräftigem Rühren und unter Ausschluß von Sauerstoff in einer Stickstoffatmosphäre im Temperaturbereich von 0 bis 5 °C innerhalb von 6 Stunden tropfenweise ein Gemisch aus 45 g (1 Mol) Formamid und 1,5 g (0,01 Mol) Trifluormethansulfonsäure zugegeben. Anschließend rührt man die Reaktionsmischung eine Stunde bei 10 °C und danach 20 Stunden bei 20 °C weiter. Durch Zugabe von n-Tributyl-amin wird das Reaktionsgemisch neutralisiert. Flüchtige Anteile des Reaktionsgemisches werden im Vakuum der Wasserstrahlpumpe abdestilliert, wobei das Gemisch bis höchstens 25 °C erhitzt wird. Das verbleibende Öl wird anschließend fraktioniert destilliert, wobei man 57 g (55 % der Theorie), bezogen auf Formamid, an N-α-Methoxy-ethyl-formamid vom Siedepunkt 59 bis 62 °C bei einem Druck von 0,5 mbar erhält.

## Beispiel 3

Beispiel 2 wird mit der Ausnahme wiederholt, daß man anstelle von Trifluormethansulfonsäure als saurem Katalysator nunmehr 1,6 g (0,01 Mol) p-Toluolsulfonsäure einsetzt. Bezogen auf Formamid erhält man in 45 %iger Ausbeute N-α-Methoxy-ethyl-formamid.

## Beispiel 4

In einem Dreihalskolben, der mit Thermometer, Rückflußkühler und Tropftrichter ausgestattet ist, legt man 72 g (1 Mol) Vinylethylether vor, erhitzt die Vorlage auf eine Temperatur von 35° und gibt dazu innerhalb von 5 Stünden bei dieser Temperatur unter kräftigem Durchmischen eine Lösung aus 22,5 g (0,5 Mol) Formamid und 3 g (0,018 Mol) p-Toluolsulfonsäure zu. Anschließend rührt man das Reaktionsgemisch noch 2 Stunden bei einer Temperatur von 35 °C, setzt 5 g Soda zu, filtriert die Salze ab und engt die Lösung im Wasserstrahlpumpenvakuum ein. Der verbleibende ölige Rückstand wird dann über eine 30 cm lange Kolonne fraktionierend destilliert, Man erhält 46 g (79 % der Theorie), bezogen auf Formamid, an N-α-Ethoxy-ethyl-formamid vom Siedepunkt 49 bis 52 °C bei einem Druck von 0,1 mbar.

## Beispiel 5

Zu 72 g (1 Mol) Vinylethylether tropft man unter kräftigem Rühren und unter Ausschluß von Sauerstoff in einer Stickstoffatmosphäre ein Gemisch aus 45 g (1 Mol) Formamid und 3 g (0,011 Mol) Zinn-(IV)-chlorid bei einer Temperatur von 35 °C innerhalb von 7 Stunden zu. Nach Zugabe des Gemisches aus Formamid und Katalysator wird das Reaktionsgemisch noch 5 Stunden bei einer Temperatur von 40 °C gerührt. Danach destilliert man im Wasserstrahlpumpenvakuum die flüchtigen Anteile ab, nimmt den Rückstand in 300 ml Chloroform auf und wäscht die resultierende Lösung zweimal mit je 150 ml einer gesättigten Kochsalzlösung. Die organische Phase wird anschließend über Natriumsulfat getrocknet. Danach destilliert man das Lösemittel ab und fraktioniert den Rückstand unter einem Druck von 0,1 mbar. Man erhält N-α-Ethoxy-ethyl-formamid in einer Ausbeute von 53 % der Theorie, bezogen auf das eingesetzte Formamid.

Beispiel 6

In einem Dreihalskolben, der mit Thermometer, Rückflußkühler und Tropftrichter ausgestattet ist, legt man 150 g (2,5 Mol) Vinylmethylether in 100 g Tetrahydrofuran bei einer Temperatur von 5 °C vor. Bei dieser Temperatur wird unter Stickstoffatmosphäre ein Gemisch aus 90 g (2 Mol). Formamid, 3,6 g (0,045 Mol) absolutem Pyridin und 80 g Tetrahydrofuran innerhalb von 7 Stunden zugetropft. Man beläßt anschließend die Reaktionsmischung 1 Stunde bei 5 °C und rührt danach 48 Stunden bei 20 °C weiter.

Man stellt danach durch Zugabe von Eisessig einen pH-Bereich von 7 bis 6,5 ein und destilliert flüchtige Anteile im Wasserstrahlvakuum bei einer Temperatur bis zu 25 °C ab.

Durch fraktionierende Vakuumdestillation erhält man 84 g (41 % der Theorie), bezogen auf Formamid, an N-α-Methoxy-ethyl-formamid.

**Patentansprüche**

1. Verfahren zur Herstellung von N-α-Alkoxy-ethyl-formamiden der Formel

$$CH_3 - \overset{\displaystyle OR}{\overset{|}{C}H} - NH - CHO \qquad (I)$$

in der R = $C_1$- bis $C_{18}$-Alkyl bedeutet, dadurch gekennzeichnet, daß man einen Vinylether der Formel

$$CH_2{=}CH{-}OR \qquad (II)$$

in der R = $C_1$- bis $C_{18}$-Alkyl bedeutet, in Gegenwart eines sauren oder basischen Katalysators bei Temperaturen von —10 bis 150 °C mit Formamid in einem molaren Verhältnis von 20 : 1 bis 1 : 2 umsetzt, wobei die Menge des Katalysators 0,1 bis 10 Gew. % bezogen auf Vinylether und Formamid, beträgt.

2. Verfahren nach Anspruch 1, dadurch gekennzeichnet, daß man die Umsetzung in Gegenwart eines sauren Katalysators bei Temperaturen von 0 bis 70 °C durchführt.

3. Verfahren nach den Ansprüchen 1 bis 2, dadurch gekennzeichnet, daß man als sauren Katalysator Schwefelsäure, Amidosulfonsäure, aliphatische und aromatische Sulfonsäuren und/oder Lewis-Säuren einsetzt.

4. Verfahren nach den Ansprüchen 1 bis 3, dadurch gekennzeichnet, daß man als basischen Katalysator tertiäre Amine einsetzt.

5. Verfahren nach den Ansprüchen 1 bis 4, dadurch gekennzeichnet, daß man Vinylether der Formel II einsetzt, in der der Substituent R eine $C_1$- bis $C_4$-Alkylgruppe bedeutet.

6. Verfahren nach den Ansprüchen 1 bis 5, dadurch gekennzeichnet, daß man Vinylmethylether mit Formamid im Molverhältnis 3 : 1 bis 1 : 1 in Gegenwart von 0,5 bis 5 Gew. % Bortrifluoriddiethyletherat als saure Katalysator umsetzt.

7. Verwendung der nach den Ansprüchen 1 bis 6 hergestellten N-α-Alkoxy-ethyl-formamide als Einsatzstoffe zur Herstellung von N-Vinylformamid durch Pyrolyse, wobei aus den Verbindungen der Formel I R-OH eliminiert wird.

**Claims**

1. A process for the preparation of an N-α-alkoxyethylformamide of the formula

$$CH_3 - \overset{\displaystyle OR}{\overset{|}{C}H} - NH - CHO \qquad (I)$$

where R is $C_1$-$C_{18}$-alkyl, wherein a vinyl ether of the formula

$$CH_2{=}CH{-}OR$$

where R is $C_1$-$C_{18}$-alkyl, is reacted with formamide in a molar ratio of from 20 : 1 to 1 : 2 in the presence of an acidic or basic catalyst at from — 10 to 150 °C, the amount of catalyst being from 0.1 to 10 % by weight, based on vinyl ether and formamide.

2. A process as claimed in claim 1, wherein the reaction is carried out in the presence of an acidic catalyst at from 0 to 70 °C.

3. A process as claimed in either of claims 1 and 2, wherein sulfuric acid, amidosulfonic acid, an aliphatic or aromatic sulfonic acid and/or a Lewis acid are used as acidic catalysts.

4. A process as claimed in any of claims 1 to 3, wherein a tertiary amine is used as the basic catalyst.

5. A process as claimed in any of claims 1 to 4, wherein a vinyl ether of the formula II, where R is $C_1$-$C_4$-alkyl, is used.

6. A process as claimed in any of claims 1 to 5 wherein vinyl methyl ether is reacted with formamide in a molar ratio of from 1 : 3 to 1 : 1 in the presence of from 0.5 to 5 % by weight of boron trifluoride diethyl etherate, as the acidic catalyst.

7. Use of an N-α-alkoxyethylformamide prepared as claimed in any of claims 1 to 6 as a starting material for preparing an N-vinylformamide by pyrolysis with elimination of R-OH from the compound of the formula I.

**Revendications**

1. Procédé pour la préparation de N-α-alcoxyéthylformamides de formule

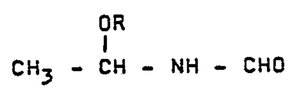

(I)

dans laquelle R représente un radical alkyle en $C_1$ à $C_{18}$, caractérisé en ce qu'on fait réagir un éther vinylique de formule

$$CH_2{=}CH{-}OR \qquad (II)$$

dans laquelle R représente un radical alkyle en $C_1$ à $C_{18}$, avec le formamide dans un rapport molaire de 20 : 1 à 1 : 2, en présence d'un catalyseur acide ou basique à des températures de — 10 à 150 °C, la quantité de catalyseur se situant entre 0,1 et 10 % en poids sur la base de l'éther vinylique et du formamide.

2. Procédé selon la revendication 1, caractérisé en ce qu'on mène la réaction en présence d'un catalyseur acide à des températures de 0 à 70 °C.

3. Procédé selon la revendication 1 ou 2, caractérisé en ce qu'on utilise, en tant que catalyseur acide, l'acide sulfurique, l'acide amidosulfonique, des acides sulfoniques aliphatiques et aromatiques et/ou des acides de Lewis.

4. Procédé selon l'une quelconque des revendications 1 à 3, caractérisé en ce qu'on utilise, en tant que catalyseur basique, des amines tertiaires.

5. Procédé selon l'une quelconque des revendications 1 à 4, caractérisé en ce qu'on utilise des éthers vinyliques de formule II dans lesquels le substituant R est un groupement alkyle en $C_1$ à $C_4$.

6. Procédé selon l'une quelconque des revendications 1 à 5, caractérisé en ce qu'on fait réagir le vinylméthyléther avec le formamide dans un rapport molaire de 3 : 1 à 1 : 1 en présence de 0,5 à 5 % en poids de complexe fluorure de bore/éther diéthylique servant de catalyseur acide.

7. Utilisation des N-α-alcoxyéthylformamides préparés selon l'une quelconque des revendications 1 à 6 comme substances de départ pour la préparation de N-vinylformamide par pyrolyse, R-OH étant éliminé des composés de formule I.